# EUROPÄISCHE PATENTSCHRIFT

(11) **EP 1 499 625 B1**
(45) Veröffentlichungstag und Bekanntmachung des Hinweises auf die Patenterteilung: **03.08.2005**
(21) Anmeldenummer: 03747412.9
(22) Anmeldetag: 17.04.2003
(51) Int. Cl.: C07F 17/02, C07B 53/00

(54) **FERROCENYLLIGANDEN UND EIN VERFAHREN ZUR HERSTELLUNG SOLCHER LIGANDEN**
FERROCENYL LIGANDS AND METHOD FOR THE PRODUCTION OF SUCH LIGANDS
LIGANDS DE FERROCENYLE ET PROCEDE DE PRODUCTION ASSOCIE

(30) Priorität: 30.04.2002 DE 10219490
(43) Veröffentlichungstag der Anmeldung: 26.01.2005
(73) Patentinhaber: Degussa AG, 40474 Düsseldorf (DE)
(72) Erfinder: KNOCHEL, Paul, 81475 München (DE); LOTZ, Matthias, CH-4054 Basel (CH); MONSEES, Axel, 60487 Frankfurt (DE); RIERMEIER, Thomas, 61130 Nidderau-Ostheim (DE); KADYROV, Renat, 65931 Frankfurt (DE); ALMENA, Juan, 63450 Hanau (DE)
(86) Internationale Anmeldenummer: PCT/EP2003/004054
(87) Internationale Veröffentlichungsnummer: WO 2003/093285

(56) Entgegenhaltungen:
- DE-A- 19 952 348

## Beschreibung

Die Erfindung betrifft ausgewählte enantiomerangereicherte bidentate Organophosphor-Ferrocenylliganden, die insbesondere für die enantioselektive Hydrierung geeignet sind, sowie ein Verfahren zur Herstellung solcher Liganden.

Trisubstituierte bidentate Organophosphorverbindungen haben eine große Bedeutung als Liganden in der homogenen Katalyse erlangt. Insbesondere der Einsatz von Bisphosphinkatalysatoren in der asymmetrischen Hydrierung ist seit längerem bekannt (Burk et al., Tetrahedron 1994, 4399), wobei z. B. WO96/32400 und WO95/21151 der Einsatz von nicht C₂-symmetrischen Ferrocenyl-Liganden beschrieben ist.

Katalysatoren, die die bis dato bekannten, nicht symmetrischen Ferrocenylliganden enthielten sind allerdings nur bedingt für gezielte enantioselektive Synthesen verwendbar. In DE 199 52 348 wird demgegenüber eine neue Klasse von enantiomerangereicherten Ferrocenylliganden mit der allgemeinen Formel 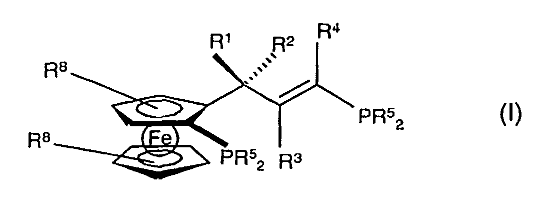 zur Verfügung gestellt, die sich insbesondere in der asymmetrischen homogenen katalytischen Hydrierung als gute enantioselektiv arbeitende Katalysatorliganden herausgestellt haben. Im Hinblick auf einen großtechnischen Einsatz ist allerdings die Suche nach Katalysatorliganden, die für eine asymmetrische Hydrierung besonders geeignet sind, weiterhin von großem Interesse. Ebenso ist die Entwicklung weiterer enantioselektiver Herstellungsverfahren mit guten Ausbeuten für solche Liganden von großer Bedeutung, um die Verfügbarkeit der Katalysatoren für eine großtechnische Prozessführung sicherzustellen.

Aufgabe der vorliegenden Erfindung war es nun Ferrocenylliganden bereitzustellen, die in der homogen-katalytischen asymmetrischen Hydrierung von ungesättigten Bindungen eine sehr hohe Enantioselektivität aufweisen und die in ausreichender Menge und mit guter Enantioselektivität herstellbar sind.

Die Aufgabe wird durch Ferrocenylliganden der allgemeinen Formel (II), die als *S*_{*fc*}-Diastrereomer (IIa) oder *R*_{*fc*}-Diastereomer (IIb) vorliegen können, 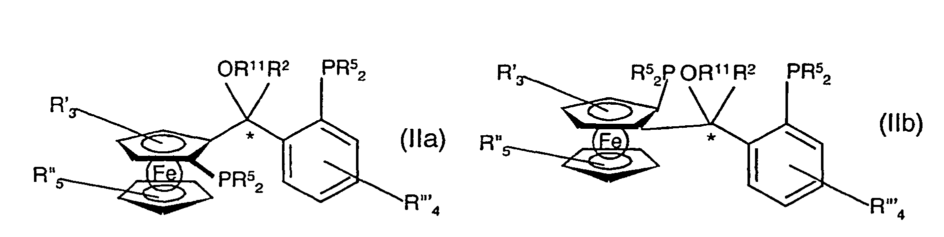 gelöst, die aus der in DE 199 52 348 beschriebenen Verbindungsklasse ausgewählt sind, wobei das *S*_{*fc*},*S*-Enantiomer Formel (IIIa) im Gemisch (IIa) oder das *R*_{*fc*},*R*-Enantiomer Formel (IIIb) im Gemisch (IIb) 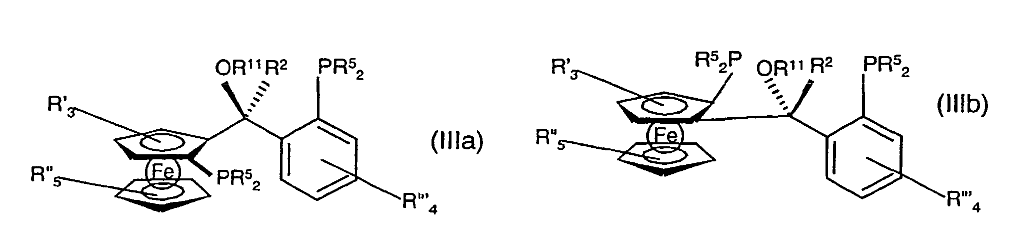 angereichert ist und
- R' und R": für Reste stehen, die unabhängig voneinander aus der Gruppe H oder CH₃ ausgewählt sein können oder die für einen Linker stehen können, der den Liganden mit einem polymeren Träger verbindet und die Reste
- R"': für Reste stehen, die unabhängig voneinander aus der Gruppe H oder (C₁-C₄)-Alkyl ausgewählt sein können und worin die Reste
- R⁵: unabhängig voneinander für einen C₆-Aryl-, C₅-C₆-Cycloalkyl-, Adamantyl- oder C₁-C₄-Alkylrest stehen, die ein oder mehrere (C₁-C₄)-Alkylsubstituenten tragen können und
- R²: für ein Wasserstoff oder einen (C₁-C₄)-Alkylrest steht und
- R¹¹: für einen (C₁-C₄)-Alkylrest steht.

Bevorzugter Alkylrest R² und R¹¹ ist ein Methylrest. Insbesondere bevorzugt sind Ferrocenylliganden der Formel (II), bei denen R', R", R''' und R² für Wasserstoffreste stehen und der Rest R¹¹ für einen Methylrest steht. Die Reste R⁵ stehen bevorzugt für Cyclohexyl-, Cyclopentyl-, Adamantyl-, Isopropyl-, tert.-Butylreste und besonders bevorzugt für Phenyl-, Toluyl- oder Xylylreste.

Die beanspruchten Liganden können weiterhin über einen geeigneten Linker R' oder R" an ein Polymer fixiert sein. Typische Linker sind z. B. Reste der Form B-X-Z, wobei X ein Spacer, wie z. B. 1,4'-Biphenyl, 1-,2-Ethylen oder 1-,3-Propylen ist und B für einen CR⁹, NR⁹, O, S, SiR⁹₂-Rest, mit R⁹ gleich H oder (C₁-C₁₈)-Alkyl, steht und Z für einen funktionelle Gruppe, wie z. B. O-, NH-, COO-, CONH-; CH2=CH-, NHCONH-, OCONH- oder NHCOO- steht.

Weiterhin sind Ferrocenylliganden der Formel (II) bevorzugt, bei denen das angereicherte *S*_{*fc*}*,S*-Enantiomer oder *R*_{*fc*}*,R*-Enantiomer einen Anteil von 60%, bevorzugt 75%, besonders bevorzugt 90%, übersteigt. Ideal ist die Verwendung von Stereoisomeren mit einer Reinheit über 99%.

Es kann überraschend gezeigt werden, dass Katalysatorkomplexe, die Ferrocenylliganden-Stereomere der Formel (III) im Überschuss enthalten eine höhere Enantioselektivität bei der asymmetrischen Hydrierung ungesättigter Verbindungen aufweisen als die in DE 199 52 348 explizit beschriebenen, die vor allem in der *S*_{*fc*}*,R*-Enantiomer angereicherten Form vorliegen. Mit dem dort beschriebenen Herstellungsverfahren werden Ferrocenylliganden erhalten, bei denen gerade das *S*_{*fc*}*,R*-Enantiorner bzw. das *R*_{*fc*}*,S*-Enantiomer im Überschuss vorliegt.

Die beanspruchten Ferrocenylliganden, bei denen das *S*_{*fc*}*,S*-Enantiomer bzw. das *R*_{*fc*}*,R*-Enantiomer angereichert ist, können anhand der im folgenden gegebenen allgemeinen Synthesebeschreibung hergestellt werden. Hierbei ist es besonders vorteilhaft, dass ohne weiteren Anreicherungsschritt bereits das *S*_{*fc*}*,S*-Enantiomer oder das *R*_{*fc*}*,R*-Enantiomer im Überschuss gebildet wird.

Ein solches Verfahren wird im folgenden exemplarisch am Beispiel der Synthese der *S*_{*fc*}*,S*- Enantiomere beschrieben. Im ersten Herstellungsschritt wird ein Ferrocen nach einer Methode von *Kagan et al.* (*J. Org. Chem.* **1995,** *60,* 2502) zum chiralen Ferrocenyl-sulfoxid **1** umgesetzt. Zur Einführung der aromatischen Gruppe und der ersten phosphorhaltigen Gruppe wird in Gegenwart einer Lithiumbase der Ferrocenylring lithiiert und transmetalliert. In Gegenwart eines Metallkatalysators der 8. Nebengruppe, insbesondere eines Rhodiumkatalysators findet die Kupplung zu der entsprechenden Verbindung **2** statt, wobei das Stereoisomer **2a** im Überschuss entsteht.

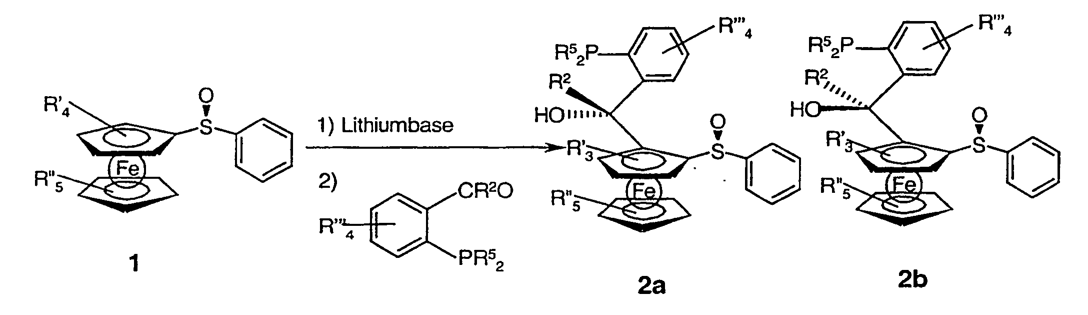

Die erhaltene Enantiomerenmischung kann direkt oder in einer alternativen Ausführungsform erst getrennt und anschließend weiterverarbeitet werden. Im folgenden werden die Reaktionsschemen anhand der Umsetzung des Enantiomers **2a** wiedergegeben. Die Verbindung **2a** wird anschließend nach Zugabe eines Alkalihydrids, wie z. B. KH, mit dem entsprechenden lodid zur Verbindung **3a** umgesetzt.

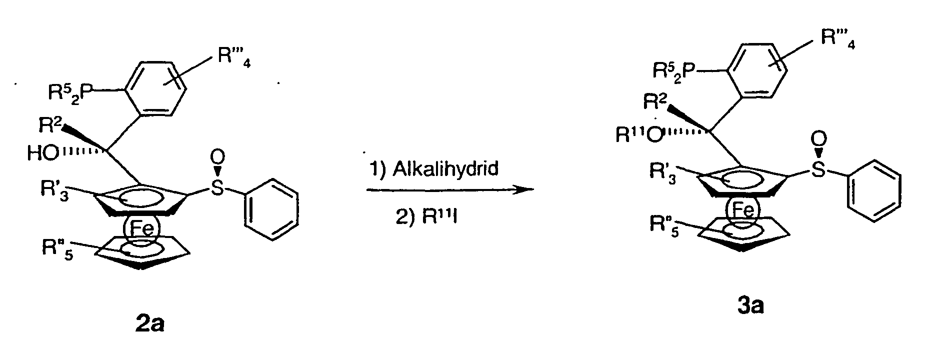

Die Sulfoxidgruppe lässt sich in Gegenwart einer starken Lithiumbase durch die zweite phosphorhaltige Gruppe substituieren. Man gelangt so zu dem erfindungsgemäßen Liganden, wobei das *S*_{*fc*}*,S*-Enantiomer **4a** im Überschuss vorliegt.

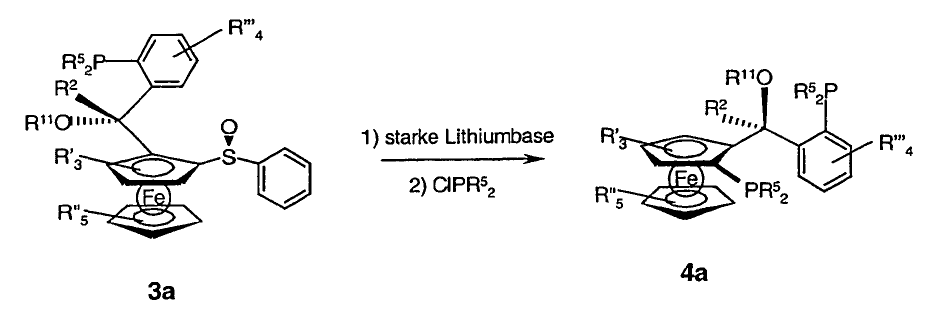

Die Verbindung **2b** wird zu den entsprechenden Verbindungen **3b** und **4b** umgesetzt. Durch Trennung der Diastereomere **2a** und **2b** und/oder der Diastereomere **3a** und **3b** kann die Enantiomerenreinheit der Synthese weiter gesteigert werden.

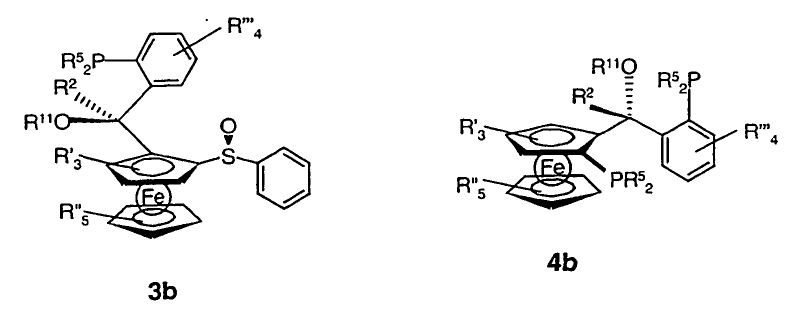

Mit dem beschriebenen Verfahren werden direkt Diastereomerengemische erhalten, die sich durch einen hohen Anteil an *S*_{*fc*}*,S*-Enantiomeren auszeichnen, wobei die erhaltenen Gemische direkt als Liganden für Katalysatoren eingesetzt werden können. Durch weitere Aufreinigung, z. B. mit chromatographischen Verfahren, kann ein noch höherer Überschuss des *S*_{*fc*}*,S*-Enantiomers erreicht werden.

Entsprechend führt die Verwendung des *R*-Enantiomereduktes zu den enantiomeren *R*_{*fc*}*,R*-Produkten im Überschuss.

Das beschriebene Verfahren kann nicht nur zur enantioselektiven Synthese der besonders für die asymmetrische Hydrierung geeigneten Liganden genutzt werden, sondern ist darüber hinaus zur Synthese von Ferrocenylliganden der allgemeinen Formel (II) geeignet, wobei das *S*_{*fc*}*,S*-Enatiomer bzw. das *R*_{*fc*}*,R*-Enantiomer der Formel (III) im Überschuss vorliegt.

Somit betrifft, die vorliegende Erfindung weiterhin ein Verfahren zur Herstellung von *S*_{*fc*}*-* oder *R*_{*fc*}-Ferrocenylliganden der Formel (II), 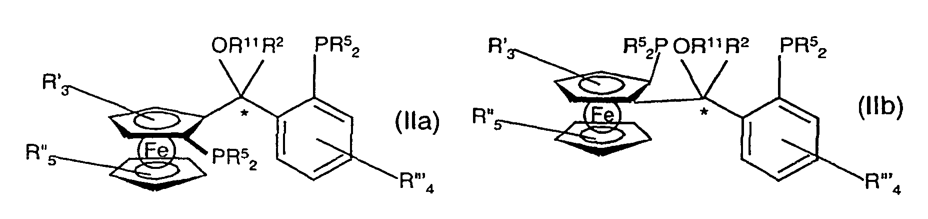 wobei das *S*_{*fc*}*,S*-Enantiomer Formel (IIIa) im Gemisch (IIa) oder das *R*_{*fc*}*,R*-Enantiomer Formel (IIIb) im Gemisch (IIb) 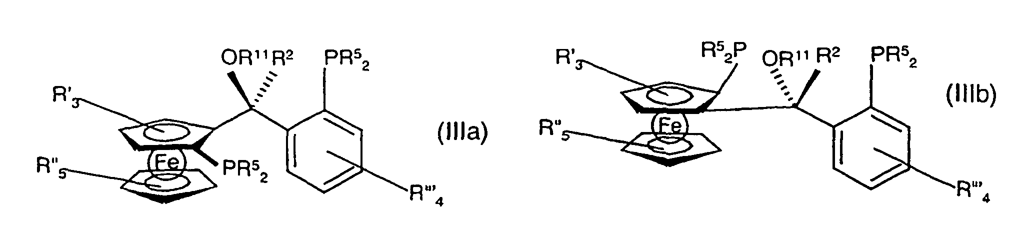 angereichert ist und worin
- R' und R": unabhängig voneinander für einen Substituenten stehen kann, der aus der Gruppe H und (C₁-C₄)-Alkyl ausgewählt ist oder für einen Linker steht, der den Liganden mit einem Polymer verbindet und die Reste
- R''': unabhängig voneinander aus der Gruppe H, (C₁-C₁₈)-Alkyl, (C₁-C₁₈)-Alkoxy, (C₁-C₁₈)-Acyloxy, (C₆-C₁₄)-Aryl, (C₃-C₁₈)-Heteroaryl, (C₂-C₁₇)-Heteroalkyl, (C₃-C₈)-Cycloalkyl, (C₂-C₁₀)-Alkenyl ausgewählt sein, wobei auch zwei benachbarte Reste zu einem Ringsystem miteinander verknüpft sein können und die Reste
- R⁵: unabhängig voneinander (C₁-C₁₈)-Alkyl, (C₆-C₁₈)-Aryl, (C₆-C₁₈)-Aryl-(C₁-C₈)-Alkyl, (C₃-C₁₈)-Heteroaryl, (C₃-C₁₈)-Heteroaryl-(C₁-C₈)-Alkyl, (C₂-C₁₇)-Heteroalkyl, (C₃-C₈)-Cycloalkyl, (C₃-C₈)-Cycloalkyl-(C₁-C₈)-Alkyl, (C₂-C₁₀)-Alkenyl sein können, die ein oder mehrere (C₁-C₄)-Alkylsubstituenten tragen können und der Rest
- R²: ein H oder ein (C₁-C₈)-Alkylrest, (C₆-C₁₈)-Aryl, (C₆-C₁₈)-Aryl-(C₁-C₈)-Alkylrest sein kann und worin der Rest
- R¹¹: ein (C₁-C₁₈)-Alkyl, (C₆-C₁₈)-Aryl, (C₆-C₁₈)-Aryl-(C₁-C₈)-Alkylrest sein kann.
umfassend folgende Verfahrensschritte:
a) Kupplung von chiralem Ferrocenyl-sulfoxid mit einem aromatischen Aldehyd der Formel (IV), 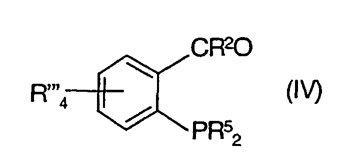 wobei das chirale Ferrocenyl-sulfoxid in Gegenwart einer Lithiumbase lithiiert wird und anschließend die Kupplung des aromatischen Aldehyds durch Transmetallierung in Gegenwart eines Metallkatalysators der 8. Nebengruppe durchgeführt wird,
b) Kopplung der freien OH-Gruppe am chiralen Zentrum des Reaktionsproduktes aus Schritt a) mit einem organischen Rest R¹¹ durch Zugabe eines Halogenids R¹¹Hal, bevorzugt des lodids R¹¹I, in Gegenwart eines Alkalihydrids,
c) Substitution der Sulfoxidgruppe des Reaktionsproduktes aus Schritt b) in Gegenwart einer starken Lithiumbase durch ein Phosphohalogenid der Formel HalPR⁵₂, bevorzugt durch ein Phosphochlorid CIPR⁵₂.

Bevorzugte eignet sich das beschriebenen Verfahren zur Herstellung von Liganden, die als Reste R', R", R''' unabhängig voneinander H und/oder CH₃ tragen. Die Reste R⁵ stehen bevorzugt unabhängig voneinander für C₆-Aryl-, (C₅-C₆)-Cycloalkyl-, Adamantyl- oder (C₁-C₄)-Alkylreste. R² steht bevorzugt für ein H oder eine (C₁-C₄)-Alkylgruppe und R¹¹ für eine (C₁-C₄)-Alkylgruppe.

Die beanspruchten Liganden können einfach mit Metallen zu den entsprechenden Komplexverbindungen umgesetzt werden. Typischerweise finden die beanspruchten Liganden in komplexierter Form Verwendung in der asymmetrischen Hydrierung von ungesättigten Verbindungen, wobei sie mit Metallen Komplexverbindungen bilden, die als Katalysatoren eingesetzt werden können. Die Katalysatoren können entweder direkt in einer Ein-Topf-Reaktion durch einfaches zusammengeben von erfindungsgemäßem Ligand und Metall, Metallsalz oder Metallvorkomplex intermediär gebildet werden oder auch vorab hergestellt und isoliert und dem Reaktionsansatz als fertige Komplexverbindung zugegeben werden. Als Koordinationszentrum kommen insbesondere die Metalle der 7. und 8. Nebengruppe, bevorzugt Co, Ni, Ru, Pd, Ir, Pt, besonders bevorzugt Rh in Frage, wobei Komplexverbindungen gebildet werden, die zumindest einen erfindungsgemäßen Liganden enthalten. Daneben können weitere Liganden im Komplex vorhanden sein, die z. B. aus dem Vorkomplex, dem Lösemittel oder einem sonstigen Reaktionszusatz stammen können.

Beispiele für Metallsalze, die zur Herstellung der Komplexverbindungen geeignet sind, sind Metallchloride, -bromide, -iodide, -cyanide, -nitrate, -acetate, - acetylacetonate, -hexafluoracetylacetonate, tetrafluoroborate, -perfluoracetate oder -triflate, insbesondere des Palladium, Platins, Rhodium, Ruthenium, Iridium, Kobalts oder/und Nickels.

Beispiele für geeignete Vorkomplexe sind: Cyclooctadienpalladiumchlorid, Cyclooctadienpalladiumiodid, 1,5-Hexadienpalladiumchlorid, 1,5-Hexadienpalladiumiodid, Bis(dibenzylidenaceton)palladium, Bis(acetonitril)palladium(II)chlorid, Bis(acetonitril)palladium(II)bromid, Bis(benzonitril)palladium(II)chlorid, Bis(benzonitril)palladium(II)bromid, Bis(benzonitril)palladium(II)iodid, Bis(allyl)palladium, Bis(methallyl)palladium, Allylpalladiumchlorid-Dimer, Methallylpalladiumchlorid-Dimer, Tetramethylethylendiaminpalladiumdichlorid, Tetramethylethylendiaminpalladiumdibromid, Tetramethylethylendiaminpalladiumdiiodid, Tetramethylethylendiaminpalladiumdimethyl, Cyclooctadienplatinchlorid, Cyclooctadienplatiniodid, 1,5-Hexadienplatinchlorid, 1,5-Hexadienplatiniodid, Bis(cyclooctadien)platin, Kalium(ethylentrichloroplatinat), Cyclooctadienrhodium(I)chlorid-Dimer, Norbornadienrhodium(I)chlorid-Dimer, 1,5-Hexadienrhodium(I)chlorid-Dimer, Tris(triphenylphosphan)rhodium(I)chlorid, Hydridocarbonyltris(triphenylphosphan)rhodium(I)chlorid, Bis(cyclooctadien)rhodium(I)perchlorat, Bis(cyclooctadien)rhodium(I)tetrafluorborat, Bis(cyclooctadien)rhodium(I)triflat, Bis(acetonitrilcyclooctadien)rhodium(I)perchlorat, Bis(acetonitrilcyclooctadien)rhodium(I)tetrafluorborat, Bis(acetonitrilcyclooctadien)rhodium(I)triflat, Cyclopentadienrhodium(III)chlorid-Dimer, Pentamethylcyclopentadienrhodium(III)chlorid-Dimer, (cyclooctadien)Ru(µ³-allyl)₂, ((cyclooctadien)Ru)₂(acetat)₄, ((Cyclooctadien)Ru)₂(trifluoracetat)₄, RuCl₂(Aren)-Dimer, Tris(triphenylphosphan)ruthenium(II)chlorid, Cyclooctadienruthenium(II)chlorid, Cyclooctadieniridium(I)chlorid-Dimer, Bis(cycloocten)iridium(I)chlorid-Dimer, Bis(cyclooctadien)nickel, (Cyclododecatrien)nickel, Tris(norbornen)nickel, Nickeltetracarbonyl, Nickel(II)acetylacetonat.

Ein weiterer Gegenstand der vorliegenden Erfindung ist die Verwendung der beanspruchten Ferrocenylliganden und der Katalysatoren, die solche Liganden enthalten zur asymmetrischen Hydrierung von ungesättigten organischen Verbindungen. Dabei zeigen solche Katalysatoren eine höhere Enantioselektivität. Besonders geeignet sind die erfindungsgemäßen Komplexe bei der asymmetrischen Hydrierung von C=C-, C=O- oder C=N-Bindungen, in denen sie hohe Aktivitäten und Selektivitäten aufweisen sowie in der asymmetrischen Hydroformylierung.

### Ausführungsbeispiele

### Beispiel 1: Darstellung von einzelnen Verbindungen der Formel (2a) und (2b)

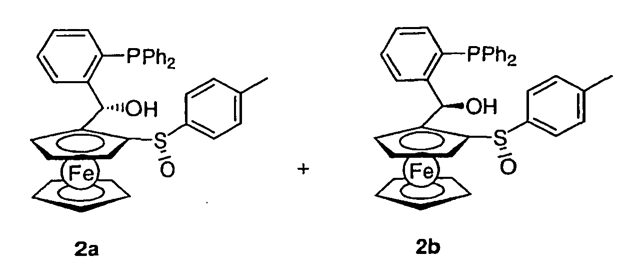

In einem 100 ml Rundkolben mit Argoneinlass wurde (*S*)-Ferrocenyl-*p*-tolylsulfoxid (1) (1.78 g, 5.49 mmol) in THF (40 ml) gelöst und die Lösung auf -78 °C gekühlt. Im Anschluss wurde langsam Lithiumdiisopropylamin-Lösung (3.30 ml, 6.59 mmol; 2.0 M in THF) zugegeben. Nach 60 minütigem Rühren bei -78 °C wurde tropfenweise 2-(Diphenylphosphino)-benzaldehyd (2.08 g, 7.14 mmol) gelöst in THF (5 ml) zugegeben und die Reaktionslösung 1 h lang bei -78 °C gerührt. Nach Rühren über Nacht bei Raumtemperatur wurde mit ges. NH₄Cl-Lösung (20 ml) gequencht, die organische Phase abgetrennt und die wässrige Phase mit CH₂Cl₂ (2 mal 50 ml) extrahiert. Die vereinigten organischen Phasen wurden mit NaCl-Lösung (20 ml) gewaschen, über MgSO₄ getrocknet, abfiltriert und das Lösemittel am Rotationsverdampfer abdestilliert. Das Rohprodukt wurde säulenchromatographisch gereinigt (*n*-Pentan/Diethylether 2:1). Der Alkohol **2a** (1.32 g, 2.14 mmol, 39 %) (Schmp.: 159 °C) und der Alkohol **2b** (0.99 g, 1.61 mmol, 29 %) wurden als gelbe Feststoffe erhalten. Des weiteren konnte ein Teil des eingesetzten Sulfoxids **1** (429 mg, 1.32 mmol, 24 %) zurückgewonnen werden.

### 2a:

[α]D²⁰ = + 465.20 (c = 0.87, CHCl₃).
IR (KBr): 3436 (br, vs), 3053 (m), 2922 (m), 1634 (m), 1435 (m), 1045 (m), 1011 (m), 810 (m), 745 (m), 696 (m), 505 (m).
¹H-NMR (CDCl₃, 300 MHz): 7.92-7.88 (m, 1 H), 7.41-7.37 (m, 3 H), 7.19-6.75 (m, 14 H), 6.20 (d, *J* = 0.6 Hz, 1 H), 5.91 (d, *J =* 7.8 Hz, 1 H), 4.49 (s, br, 6 H), 3.82-3.80 (m, 1 H), 3.12-3.11 (m, 1 H), 2.27 (s, 3 H).
¹³C-NMR (CDCl₃, 75 MHz): 146.57 (d, *J =* 23.3 Hz), 140.54 (d, *J =* 11.7 Hz), 137.10 (d, *J =* 11.7 Hz), 136.11 (d, *J =* 11.6 Hz), 134.91-132.84 (m), 129.79, 128.93-126.80 (m), 124.08, 94.37, 88.75, 74.75 (d, *J*=2.9 Hz), 70.40, 70.16, 66.76, 65.16 (d, *J* 30.4 Hz), 21.35.
³¹P-NMR (CDCl₃, 81 MHz): -17.26.
MS (EI): 614 (M⁺, 9), 598 (46), 597 (42), 533 (33), 475 (11), 459 (19), 398 (19), 353 (43), 337 (100), 308 (33), 290 (29), 261 (51), 183 (53), 124 (51), 91 (56), 77 (14).

| | | | |
|---|---|---|---|
| C₃₆H₃₁FeO₂PS (614.53) | HR-MS | Ber. | 614.1132. |
| | | Gef. | 614.1137. |

### 2b:

IR (KBr): 3436 (br, vs), 3053 (m), 2923 (m), 1636 (m), 1435 (s), 1026 (s), 1011 (s), 810 (m), 745 (s), 697 (s), 503 (s).
³¹P-NMR (CDCl₃, 81 MHz): -14.77.
MS (EI): 614 (M⁺, 11), 598 (75), 597 (46), 533 (70), 475 (15), 459 (53), 398 (33), 353 (46), 337 (100), 306 (19), 259 (23), 183 (32), 124 (56), 91 (64), 77 (19).

| | | | |
|---|---|---|---|
| C₃₆H₃₁FeO₂PS (614.53) | HR-MS | Ber. | 614.1132. |
| | | Gef. | 614.. |

### Beispiel 2: Darstellung einer Verbindung der Formel (3a)

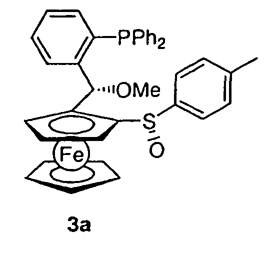

In einem 50 ml-Rundkolben mit Argoneinlass wurde KH (41 mg, 1.02 mmol) in THF (1 ml) suspendiert und auf 0 °C gekühlt. Anschließend wurde langsam der Alkohol **2a** aus Bsp.1 (482 mg, 0.78 mmol) in THF (9 ml) gelöst bei 0 °C zugetropft und das Reaktionsgemisch 1 h bei Raumtemperatur gerührt . Es wurde erneut auf 0 °C gekühlt und tropfenweise Mel (122 mg, 0.86 mmol) zugegeben. Nach 10 minütigem Rühren bei 0 °C und 30 minütigem Rühren bei Raumtemperatur wurde mit ges. NH₄Cl-Lösung (20 ml) gequencht, die organische Phase abgetrennt und die wässrige Phase mit CH₂Cl₂ (2 mal 50 ml) extrahiert. Die vereinigten organischen Phasen wurden mit NaCl-Lösung (20 ml) gewaschen, über MgSO₄ getrocknet, abfiltriert und das Lösemittel am Rotationsverdampfer abdestilliert. Das Rohprodukt wurde säulenchromatographisch gereinigt (*n*-Pentan/Et₂O 1:1, dann Et₂O). Der Methylether **3a** (355 mg, 0.56 mmol, 72 %) wurde als gelber Feststoff (Schmp.: 98 - 100 °C) erhalten.
[α]D²⁰ = -28.20 (c = 0.61, CHCl₃).
IR (KBr): 3053 (m), 2925 (m), 1631 (m), 1435 (m), 1087 (s), 1042 (s), 817 (m), 746 (s), 697 (s), 545 (m), 500 (s).
¹H-NMR (CDCl₃, 300 MHz): 7.53-7.50 (m, 2 H), 7.46-7.42 (m, 1 H), 7.27-7.09 (m, 14 H), 7.00-6.95 (m, 1 H), 6.17 (d, *J* = 7.2 Hz, 1 H), 4.14 (s, 5 H), 3.99-3.97 (m, 1 H), 3.85-3.84 (m, 2 H), 3.17 (s, 3 H), 2.30 (s, 3 H).
³¹P-NMR (CDCl₃, 81 MHz): -17.45.
MS (EI): 628 (M⁺, 16), 612 (11), 563 (31), 531 (22), 489 (8), 459 (11), 353 (45), 337 (100), 261 (4), 183 (24), 121 (11), 91 (13).

| | | | |
|---|---|---|---|
| C₃₇H₃₃FeO₂PS (628.55) | HR-MS | Ber. | 628.1288. |
| | | Gef. | 628.1306. |

### Beispiel 3: Darstellung einer Verbindung der Formel (3b)

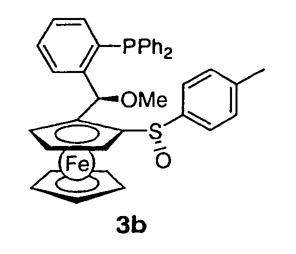

In einem 50 ml-Rundkolben mit Argoneinlass wurde KH (37 mg, 0.92 mmol) in THF (1 ml) suspendiert und auf 0 °C gekühlt. Anschließend wurde langsam der Alkohol **2b** aus Bsp.1 (433 mg, 0.70 mmol) in THF (9 ml) gelöst bei 0 °C zugetropft und das Reaktionsgemisch 1 h bei Raumtemperatur gerührt . Es wurde erneut auf 0 °C gekühlt und tropfenweise Mel (111 mg, 0.78 mmol) zugegeben. Nach 10 minütigem Rühren bei 0 °C und 10 minütigem Rühren bei RT wurde mit ges. NH₄Cl-Lösung (20 ml) gequencht, die organische Phase abgetrennt und die wässrige Phase mit CH₂Cl₂ (2 mal 50 ml) extrahiert. Die vereinigten organischen Phasen wurden mit NaCl-Lösung (20 ml) gewaschen, über MgSO₄ getrocknet, abfiltriert und das Lösemittel am Rotationsverdampfer abdestilliert. Das Rohprodukt wurde säulenchromatographisch gereinigt (*n*-Pentan/Et₂O 1:3). Der Methylether **3b** (385 mg, 0.61 mmol, 88 %) wurde als gelber Feststoff (Schmp.: 110-112 °C) erhalten.
IR (KBr): 3053 (m), 2925 (m), 1636 (m), 1434 (m), 1084 (s), 1044 (s), 813 (m), 746 (s), 697 (s), 498 (s).
³¹P-NMR (CDCl3, 81 MHz): -14.67.

| | | | |
|---|---|---|---|
| C₃₇H₃₃FeO₂PS (628.55) | HR-MS | Ber. | 628.1288. |
| | | Gef. | 628.. |

### Beispiel 4: Darstellung einer Verbindung der Formel (4a)

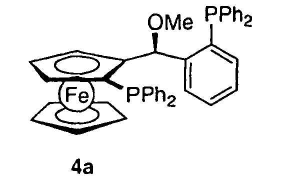

In einem 50 ml-Rundkolben mit Argoneinlass wurde Verbindung **3a** aus Bsp.2 (155 mg, 0.25 mmol) in THF (3 ml) gelöst und die Lösung auf -78 °C gekühlt. Im Anschluss wurde langsam *t*-BuLi ( 0.31 ml, 0.49 mmol, 1.6 M in Hexan) zugetropft und 10 min. bei -78 °C gerührt. Es wurde tropfenweise Chlordiphenylphosphan (0.15 ml, 0.86 mmol) zugegeben, das Kühlbad entfernt und das Reaktionsgemisch 30 min. bei Raumtemperatur gerührt. Nach Quenchen mit ges. NH₄Cl-Lösung (20 ml) wurde die organische Phase abgetrennt und die wässrige Phase mit Diethylether (2 mal 50 ml) extrahiert. Die vereinigten organischen Phasen wurden mit NaCl-Lösung (20 ml) gewaschen, über MgSO₄ getrocknet, abfiltriert und das Lösemittel am Rotationsverdampfer abdestilliert. Das Rohprodukt wurde säulenchromatographisch gereinigt (*n*-Pentan/Et₂O 30:1). Das Diphosphan **4a** (127 mg, 0.19 mmol, 76 %) wurde als gelber Feststoff (Schmp.: 201 °C (Zers.)) erhalten.
IR (KBr): 3068 (m), 3054 (m), 2924 (w), 1628 (w), 1478 (m), 1434 (s), 1087 (s), 818 (w), 742 (vs), 698 (vs), 498 (s), 488 (s).
³¹P-NMR (CDCl₃, 81 MHz): -17.27 (d,.*J*= 17.2 Hz), -18.39 (d, *J*= 17.2 Hz).

| | | | |
|---|---|---|---|
| C₄₂H₃₆FeOP₂ (674.54) | HR-MS | Ber. | 674.1591. |
| | | Gef. | 674.. |

### Beispiel 5: Darstellung einer Verbindung (5a)

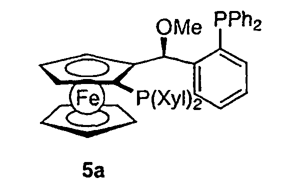

In einem 50 ml-Rundkolben mit Argoneinlass wurde Verbindung **3a** aus Bsp.2 (180 mg, 0.29 mmol) in THF (3 ml) gelöst und die Lösung auf -78 °C gekühlt. Im Anschluss wurde langsam *t*-BuLi ( 0.36 ml, 0.57 mmol, 1.6 M in Hexan) zugetropft und 10 min. bei -78 °C gerührt. Es wurde tropfenweise Chlorbis(3,5-dimethylphenyl)phosphan (277 mg, 1.00 mmol) zugegeben, das Kühlbad entfernt und das Reaktionsgemisch 30 min. bei Raumtemperatur gerührt. Nach Quenchen mit ges. NH₄Cl-Lösung (20 ml) wurde die organische Phase abgetrennt und die wässrige Phase mit Diethylether (2 mal 50 ml) extrahiert. Die vereinigten organischen Phasen wurden mit NaCl-Lösung (20 ml) gewaschen, über MgSO₄ getrocknet, abfiltriert und das Lösemittel am Rotationsverdampfer abdestilliert. Das Rohprodukt wurde säulenchromatographisch gereinigt (*n*-Pentan/Et₂O 20:1). Das Diphosphan **5a** (142 mg, 0.19 mmol, 68 %) wurde als gelber Feststoff (Schmp.: 182 °C) erhalten.
IR (KBr): 3052 (m), 2922 (m), 2818 (w), 1629 (w), 1434 (m), 1092 (m), 847 (w), 816 (w), 745 (m), 695 (s), 507 (m).
³¹P-NMR (CDCl₃, 81 MHz): -18.49 (d, *J* = 23.7 Hz), -19.05 (d, *J* = 23.7 Hz).

| | | | |
|---|---|---|---|
| C₄₆H₄₄FeOP₂ (730.65) | HR-MS | Ber. | 730.2217. |
| | | Gef. | 730.. |

### Beispiel 6: Darstellung einer Verbindung (4b)

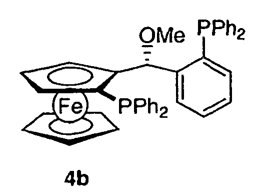

In einem 50 ml-Rundkolben mit Argoneinlass wurde Verbindung **3b** aus Bsp.3 (298 mg, 0.47 mmol) in THF (6 ml) gelöst und die Lösung auf -78 °C gekühlt. Im Anschluss wurde langsam *t*-BuLi (0.59 ml, 0.95 mmol, 1.6 M in Hexan) zugetropft und 10 min. bei -78 °C gerührt. Es wurde tropfenweise Chlordiphenylphosphan (0.30 mL, 1.66 mmol) zugegeben, das Kühlbad entfernt und das Reaktionsgemisch 30 min. bei Raumtemperatur gerührt. Nach Quenchen mit ges. NH₄Cl-Lösung (20 ml) wurde die organische Phase abgetrennt und die wässrige Phase mit Diethylether (2 mal 50 ml) extrahiert. Die vereinigten organischen Phasen wurden mit NaCl-Lösung (20 ml) gewaschen, über MgSO₄ getrocknet, abfiltriert und das Lösemittel am Rotationsverdampfer abdestilliert. Das Rohprodukt wurde säulenchromatographisch gereinigt (*n*-Pentan/Et₂O 20:1). Das Diphosphan **4b** (216 mg, 0.32 mmol, 68 %) wurde als gelber Feststoff erhalten.
IR (KBr): 3068 (m), 3052 (m), 2925 (w), 2816 (w), 1630 (w), 1479 (m), 1434 (s), 1082 (s), 820 (m), 743 (vs), 696 (vs), 501 (s), 486 (s), 456 (m).
³¹P-NMR (CDCl₃, 81 MHz): -14.69 (d,.*J*= 16.8 Hz), -20.26 (d, *J*= 16.8 Hz).

| | | | |
|---|---|---|---|
| C₄₂H₃₆FeOP₂ (674.54) | HR-MS | Ber. | 674.1591. |
| | | Gef. | 674.. |

Im folgenden werden einzelne Ausführungsbeispiele für die asymmetrische Hydrierung ungesättigter organischer Verbindungen beschrieben, wobei Liganden benutzt werden, die gemäß den Ausführungsbeispielen 1 bis 6 hergestellt wurden.

### Beispiel 7: Arbeitsvorschrift zur enantioselektiven Hydrierung von Methyl-(Z)-3-phenyl-2-methylcarboxamido-2-propenoat

### Darstellung von (S)-Methyl-2-methylcarboxamido-3-phenylpropanoat (6)

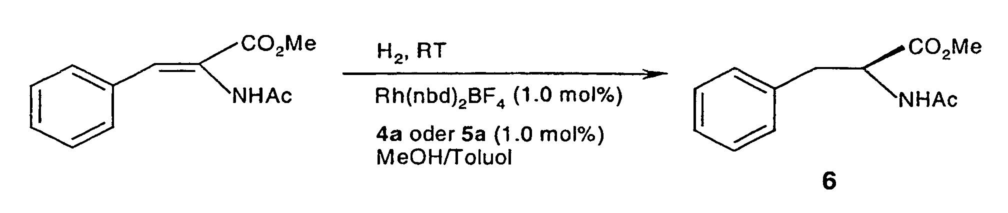

In einem 50 ml -Schlenkgetäß wurden unter Argon Rh(nbd)₂BF₄ (3.4 mg, 1 mol-%) und der Diphosphanligand (1 mol-%) in Toluol/MeOH (6 ml, 5:1) gelöst. Nachdem der Rhodiumkomplex vollständig in Lösung gegangen war, wurde Methyl-(Z)-3-phenyl-2-methylcarboxamido-2-propenoat (200 mg, 0.91 mmol) gelöst in MeOH (4 ml) zugegeben. Anschließend wurde das Schlenkgefäß über einen Dreiwegehahn mit einem Wasserstoffballon und einer Ölpumpe verbunden und die Inertgasatmosphäre durch Wasserstoff ersetzt. Die Reaktionsmischung wurde bei Raumtemperatur für die angegebene Zeit gerührt und das Lösemittel danach im Ölpumpenvakuum abkondensiert. Der Rückstand wurde durch eine kurze Silicagelsäule filtriert (Eluent: Diethylether) und das Lösemittel am Rotationsverdampfer abdestilliert. Verbindung (*S*)-**6** wurde als weißer Feststoff in quantitativer Ausbeute erhalten.
Die mit den verwendeten Liganden erzielten Enantiomerenüberschüsse sind in Tabelle 1 zusammengestellt.

**Tabelle 1:**

| Ligand* | t [h] | p [bar] | Umsatz [%] | ee [%]^{a} |
|---|---|---|---|---|
| **4a** | 1.5 | 1 | 100 | 98.5 (S) |
| **5a** | 1.5 | 1 | 100 | 98.7 (S) |
| **4b** | 2.0 | 1 | 100 | 94.2 (S) |

| | | | | |
|---|---|---|---|---|
| a) Absolute Konfiguration in Klammern | | | | |

Der Enantiomerenüberschuss wurde durch Gaschromatographie (GC) ermittelt [Säule: Chirasil-*L*-Val (0.12 µm, 25 m x 0.22 mm fused silica WCOT) der Firma Chrompack, Säulenvordruck: 83.5 kPa, Trägergas: Wasserstoff]:
GC (140 °C): tr/min = 11.61 (*R*), 12.50 (*S*).
¹H-NMR (CDCl₃, 300 MHz): 7.25-7.18 (m, 3 H), 7.04.7.00 (m, 2 H), 5.96 (d, *J =* 7.1 Hz, 1 H), 4.85-4.78 (m, 1 H), 3.65 (s, 3 H), 3.11-2.97 (m, 2 H), 1.90 (s, 3 H).
¹³C-NMR (CDCl₃, 75 MHz): 172.07, 169.52, 135.85, 129.18, 128.51, 127.06, 53.10, 52.21, 37.83, 23.02.

### Beispiel 8: Arbeitsvorschrift zur enantioselektiven Hydrierung von Dimethylitaconat

### Darstellung von 2-Methyl-succinsäuredimethylester (7)

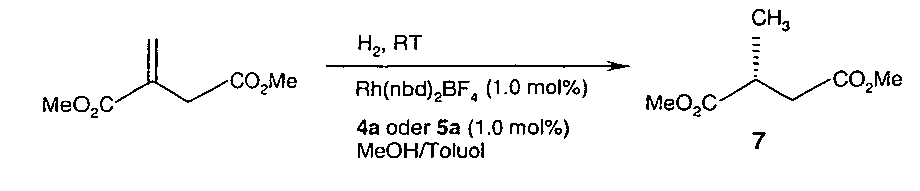

In einem 50 ml-Schlenkgefäß wurden unter Argon Rh(nbd)₂BF₄ (4.7 mg, 1 mol-%) und der Diphosphanligand (1 mol-%) in Toluol/MeOH (6 ml, 5:1) gelöst. Nachdem der Rhodiumkomplex vollständig in Lösung gegangen war, wurde Dimethylitaconat (200 mg, 1.26 mmol) gelöst in MeOH (4 ml) zugegeben. Anschließend wurde das Schlenkgefäß über einen Dreiwegehahn mit einem Wasserstoffballon und einer Ölpumpe verbunden und die Inertgasatmosphäre durch Wasserstoff ersetzt. Die Reaktionsmischung wurde bei Raumtemperatur für die angegebene Zeit gerührt und das Lösemittel danach am Rotationsverdampfer abdestilliert. Der Rückstand wurde durch eine kurze Silicagelsäule filtriert (Eluent: Diethylether). Verbindung (*R*)-7 wurde als farbloses Öl in quantitativer Ausbeute erhalten.

Die mit den verwendeten Liganden erzielten Enantiomerenüberschüsse sind in Tabelle 2 zusammengestellt.

**Tabelle 2:**

| Ligand | t [h] | p [bar] | Umsatz [%] | ee [%]^{a} |
|---|---|---|---|---|
| **4a** | 0.5 | 1 | 100 | 98.1 (R) |
| **5a** | 1.0 | 1 | 100 | 89.8 (R) |
| **4b** | 2.5 | 1 | 100 | Gering |

| | | | | |
|---|---|---|---|---|
| a) Absolute Konfiguration in Klammern | | | | |

Der Enantiomerenüberschuß wurde durch Hochleistungsflüssigkeitschromatographie (HPLC) ermittelt (HPLC-Anlage der Firma Dionex mit automatischem Probengeber und UV-VIS-Diodenarraydetektor, Säule: OD der Firma Daicel Chemical Industries, Eluent: *n*-Heptan/*i*-PrOH 95:5, flow: 0.6 ml/min, detektierte Wellenlänge: 215 nm):
HPLC (OD, 5 % *i*-PrOH, 0.6 ml/min, 215 nm): tr/min = 10.37 (*R*), 17.93 (*S*).

### Beispiel 9: Arbeitsvorschrift zur enantioselektiven Hydrierung von 2-Acetoxyacrylsäuremethylester

### Darstellung von (S)-Methyl-2-methylcarbonyloxypropanoat (8)

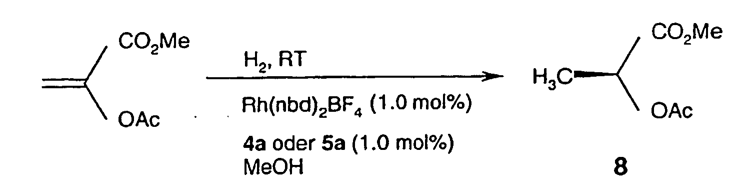

In einem 50 ml-Schlenkgefäß wurden unter Argon Rh(nbd)₂BF₄ (5.2 mg, 1 mol-%) und der Diphosphanligand (1 mol-%) in MeOH (10 ml) gelöst. Nachdem der Rhodiumkomplex vollständig in Lösung gegangen war, wurde 2-Acetoxyacrylsäuremethylester (200 mg, 1.39 mmol) zugegeben. Anschließend wurde das Schlenkgefäß über einen Dreiwegehahn mit einem Wasserstoffballon und einer Ölpumpe verbunden und die Inertgasatmosphäre durch Wasserstoff ersetzt. Die Reaktionsmischung wurde bei Raumtemperatur für 20 h gerührt und das Lösemittel danach im Ölpumpenvakuum abkondensiert. Der Rückstand wurde durch Kugelrohrdestillation gereinigt. Der Ester (*S*)-8 wurde als farbloses Öl in quantitativer Ausbeute erhalten.
Die mit den verwendeten Liganden erzielten Enantiomerenüberschüsse sind in Tabelle 3 zusammengestellt.

**Tabelle 3:**

| Ligand | t [h] | p [bar] | Umsatz [%] | ee [%]^{a} |
|---|---|---|---|---|
| **4a** | 20 | 10 | 100 | 94.9 (S) |
| **5a** | 20 | 10 | 100 | 97.3 (S) |
| **5a** | 20 | 1 | 100 | 98.5 (S) |
| **4b** | 20 | 10 | 100 | 79.8 (S) |

| | | | | |
|---|---|---|---|---|
| a) Absolute Konfiguration in Klammern | | | | |

Der Enantiomerenüberschuß wurde durch Hochleistungsflüssigkeitschromatographie (HPLC) ermittelt (HPLC-Anlage der Firma Dionex mit automatischem Probengeber und UV-VIS-Diodenarraydetektor, Säule: OD-H der Firma Daicel Chemical Industries, Eluent: *n*-Heptan/*i*-PrOH 99:1, flow: 0.6 ml/min, detektierte Wellenlänge: 215 nm):
HPLC (OD-H, 1 % *i*-PrOH, 0.6 ml/min, 215 nm): tr/min = 13.86 (S), 16.14 (*R*).
¹H-NMR (CDCl₃, 300 MHz): 4.99 (q, *J* = 7.1 Hz, 1 H), 3.65 (s, 3 H), 2.03 (s, 3 H), 1.39 (d, *J* = 7.1 Hz, 3 H).
¹³C-NMR (CDCl₃, 75 MHz): 171.26, 170.33, 68.46, 52.25, 20.58, 16.84.

Wie aus den Tabellen 1 bis 3 hervorgeht verläuft die asymmetrische Hydrierung mit den Liganden **4a** und **5a,** die beide *S*_{*fc*}*,S*-Enantiomere sind, stereoselektiver als mit den entsprechenden *S*_{*fc*}*,R*-Enantiomeren **4b.**

## Patentansprüche

1. Ferrocenylliganden der allgemeinen Formel (II) 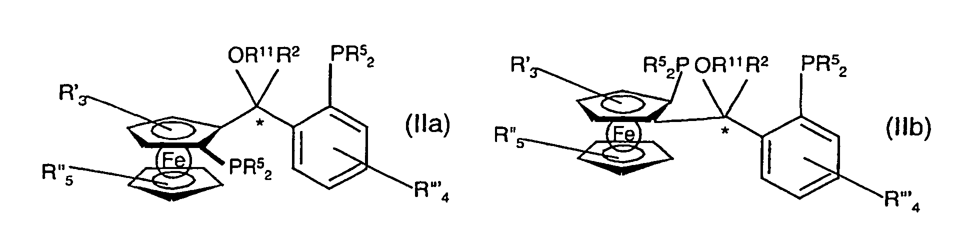 **dadurch gekennzeichnet, dass**
das *S*_{*fc*},*S*-Enantiomer Formel (IIIa) im Stereoisomerengemisch (IIa) oder das *R*_{*fc*}*,R-*Enantiomer Formel (IIIb) im Stereoisomerengemisch (IIb) 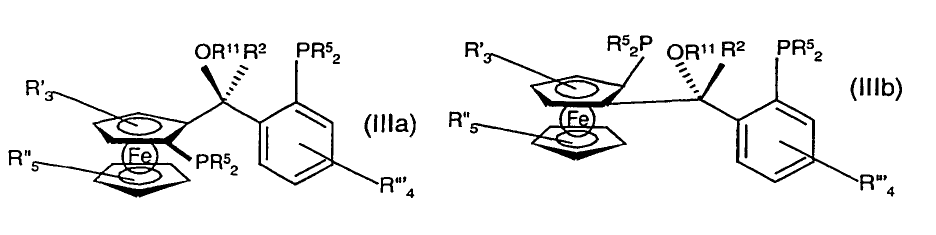 angereichert ist und
R' und R" für Reste stehen, die unabhängig voneinander aus der Gruppe H oder CH₃ ausgewählt sein können oder die für einen Linker stehen können, der den Liganden mit einem polymeren Träger verbindet und die Reste
R''' für Reste stehen, die unabhängig voneinander aus der Gruppe H oder (C₁-C₄)-Alkyl ausgewählt sein können und worin die Reste
R⁵ unabhängig voneinander für Reste ausgewählt aus der Gruppe C₆-Aryl, C₅-C₆-Cycloalkyl, Adamantyl oder C₁-C₄-Alkyl stehen können, wobei die Reste R⁵ ein oder mehrere (C₁-C₄)-Alkylsubstituenten tragen können und
R² für ein Wasserstoff oder einen (C₁-C₄)-Alkylrest steht und
R¹¹ für einen (C₁-C₄)-Alkylrest steht.

2. Ferrocenylliganden nach Anspruch 1 **dadurch gekennzeichnet, dass**
R¹¹ ein Methylrest ist und/oder
R² ein H oder ein Methylrest ist und/oder
R', R", R''' Wasserstoffreste sind und/oder die Reste
R⁵ unabhängig voneinander für Phenyl-, Toluyl- oder Xylylreste stehen.

3. Ferrocenylliganden nach einem der Ansprüche 1 oder 2 **dadurch gekennzeichnet, dass** das *S*_{*fc*},*S*-Enantiomer oder das *R*_{*fc*},*R-*Enantiomer im Stereoisomerenengemisch einen Anteil von über 60% besitzt.

4. Ferrocenylliganden nach einem der Ansprüche 1 bis 3 **dadurch gekennzeichnet, dass** der Ligand als *S*_{*fc*},*S*-Enantiomer oder als *R*_{*fc*},*R*-Enantiomer mit einer Reinheit von über 99% vorliegt.

5. Verwendung von Ferrocenylliganden nach einem der Ansprüche 1 bis 4 zur Herstellung von Komplexverbindungen.

6. Verwendung von Ferrocenylliganden nach Anspruch 5 zu Herstellung von Komplexverbindungen mit Metallen, Metallsalzen oder Metallvorkomplexen der 7. oder 8. Nebengruppe.

7. Verwendung von Ferrocenylliganden nach einem der Ansprüche 1 bis 4 bei der asymmetrischen Hydrierung oder Hydroformylierung ungesättigter organischer Verbindung.

8. Verwendung von Ferrocenylliganden nach Anspruch 7 bei der asymmetrischen Hydrierung von C=C-, C=O- oder C=N-Bindungen.

9. Verfahren zur Herstellung von Ferrocenylliganden der allgemeinen Formel (II) 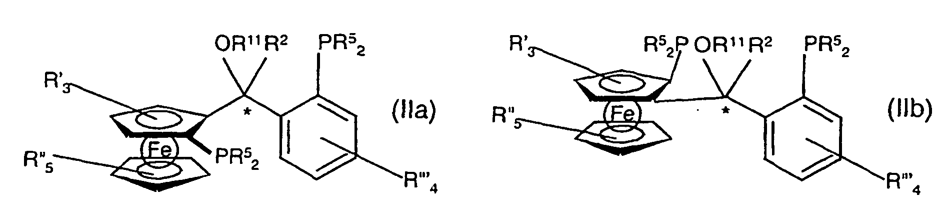 wobei das *S*_{*fc*}*,S*-Enantiomer Formel (IIIa) im Stereoisomerengemisch (IIa) oder das *R*_{*fc*}*,R*-Enantiomer Formel (IIIb) im Stereoisomerengemisch (IIb) 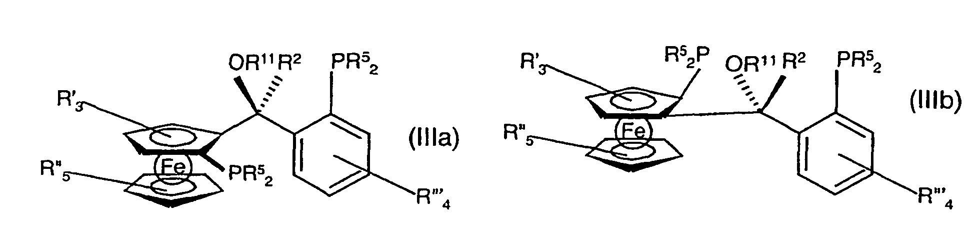 angereichert ist und worin
R' und R" unabhängig voneinander für einen Substituenten stehen kann, der aus der Gruppe H und (C₁-C₄)-Alkyl ausgewählt ist oder für einen Linker steht, der den Liganden mit einem Polymer verbindet und die Reste
R''' unabhängig voneinander aus der Gruppe H, (C₁-C₁₈)-Alkyl, (C₁-C₁₈)-Alkoxy, (C₁-C₁₈)-Acyloxy, (C₆-C₁₄)-Aryl, (C₃-C₁₈)-Heteroaryl, (C₂-C₁₇)-Heteroalkyl, (C₃-C₈)-Cycloalkyl, (C₂-C₁₀)-Alkenyl ausgewählt sein, wobei auch zwei benachbarte Reste zu einem Ringsystem miteinander verknüpft sein können und die Reste
R⁵ unabhängig voneinander (C₁-C₁₈)-Alkyl, (C₆-C₁₈)-Aryl, (C₆-C₁₈)-Aryl-(C₁-C₈)-Alkyl, (C₃-C₁₈)-Heteroaryl, (C₃-C₁₈)-Heteroaryl-(C₁-C₈)-Alkyl, (C₂-C₁₇)-Heteroalkyl, (C₃-C₈)-Cycloalkyl, (C₃-C₈)-Cycloalkyl-(C₁-C₈)-Alkyl, (C₂-C₁₀)-Alkenyl sein können, die ein oder mehrere (C₁-C₄)-Alkylsubstituenten tragen können und der Rest
R² ein H oder ein (C₁-C₈)-Alkylrest, (C₆-C₁₈)-Aryl, (C₆-C₁₈)-Aryl-(C₁-C₈)-Alkylrest sein kann und worin der Rest
R¹¹ ein (C₁-C₁₈)-Alkyl, (C₆-C₁₈)-Aryl, (C₆-C₁₈)-Aryl-(C₁-C₈)-Alkylrest sein kann,
umfassend folgende Verfahrensschritte:
a) Kupplung von chiralem Ferrocenyl-sulfoxid mit einem aromatischen Aldehyd der Formel (IV), 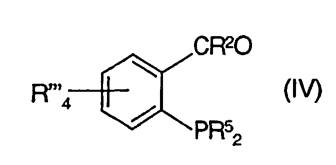 wobei das chirale Ferrocenyl-sulfoxid in Gegenwart einer Lithiumbase lithiiert wird und anschließend die Kupplung des aromatischen Aldehyds durch Transmetallierung in Gegenwart eines Metallkatalysators der 8. Nebengruppe durchgeführt wird,
b) Kopplung der freien OH-Gruppe am chiralen Zentrum des Reaktionsproduktes aus Schritt a) mit einem organischen Rest R¹¹ durch Zugabe des entsprechenden Halogenids R¹¹Hal in Gegenwart eines Alkalihydrids und
c) Substitution der Sulfoxidgruppe des Reaktionsproduktes aus Schritt b) in Gegenwart einer starken Lithiumbase durch ein Phosphohalogenids der Formel HalPR⁵₂.

10. Verfahren nach Anspruch 9 **dadurch gekennzeichnet, dass** vor der weiteren Umsetzung die erhaltenen Diastereomere aus Schritt a) und/oder die Diastereomere aus Schritt b) getrennt werden.

## Claims

1. Ferrocenyl ligands of the general formula (II) 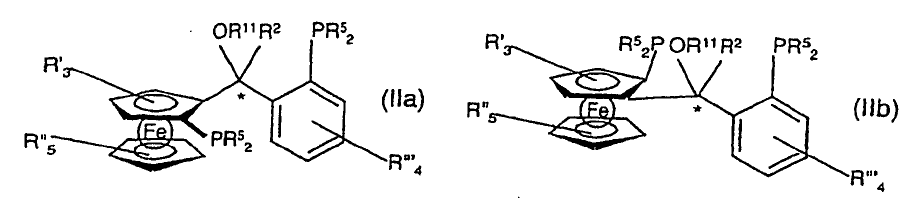 **characterized in that**
the *S*_{*fc*}*,S* enantiomer formula (IIIa) is enriched in the stereoisomer mixture (IIa) or the *R*_{*fc*}*,R* enantiomer formula (IIIb) is enriched in the stereoisomer mixture (IIb) 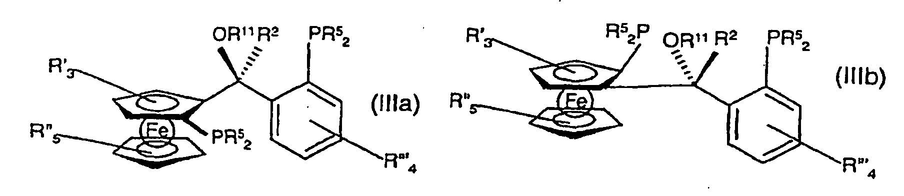 and
R' and R" represent radicals which can be chosen independently of one another from the group consisting of H or CH₃ or which can represent a linker which joins the ligand to a polymeric carrier and the radicals.
R'" represents radicals which can be chosen independently of one another from the group consisting of H or (C₁-C₄)-alkyl, and wherein the radicals
R⁵ independently of one another can represent radicals chosen from the group consisting of C₆-aryl, C₅-C₆-cycloalkyl, adamantyl or C₁-C₄-alkyl, wherein the radicals R⁵ can carry one or more (C₁-C₄)-alkyl substituents and
R² represents a hydrogen or a (C₁-C₄)-alkyl radical and
R¹¹ represents a (C₁-C₄)-alkyl radical.

2. Ferrocenyl ligands according to claim 1,
**characterized in that**
R¹¹ is a methyl radical and/or
R² is an H or a methyl radical and/or
R', R", R'" a re hydrogen radicals and/or the radicals
R⁵ independently of one another represent phenyl, toluyl or xylyl radicals.

3. Ferrocenyl ligands according to one of claims 1 or 2, **characterized in that** the *S*_{*fc*},*S* enantiomer or the *R*_{*fc*}*, R* enantiomer has a content of more than 60 % in the stereoisomer mixture.

4. Ferrocenyl ligands according to one of claims 1 to 3, **characterized in that** the ligand is in the form of the *S*_{*fc*}*,S* enantiomer or in the form of the *R*_{*fc*}*,R* enantiomer with a purity of more than 99 %.

5. Use of ferrocenyl ligands according to one of claims 1 to 4 for the preparation of complex compounds.

6. Use of ferrocenyl ligands according to claim 5 for the preparation of complex compounds with metals, metal salts or metal pre-complexes of subgroup 7 or 8.

7. Use of ferrocenyl ligands according to one of claims 1 to 4 in the asymmetric hydrogenation or hydroformylation of unsaturated organic compounds.

8. Use of ferrocenyl ligands according to claim 7 in the asymmetric hydrogenation of C=C, C=O or C=N bonds.

9. Process for the preparation of ferrocenyl ligands of the general formula (II) 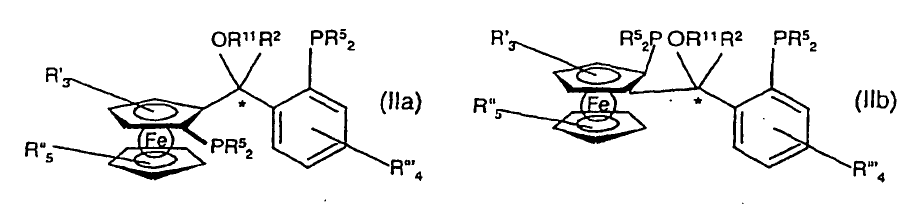 wherein the *S*_{*fc*}*,S* enantiomer formula (IIIa) is enriched in the stereoisomer mixture (IIa) or the *R*_{*fc*}*,R* enantiomer formula (IIIb) is enriched in the stereoisomer mixture (IIb) 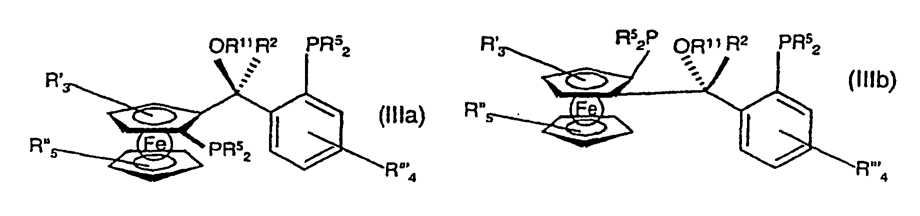 and wherein
R' and R" independently of one another can represent a substituent which is chosen from the group consisting of H and (C₁-C₄)-alkyl or represents a linker which joins the ligands with a polymer and the radicals
R'" independently of one another are chosen from the group consisting of H, (C₁-C₁₈)-alkyl, (C₁-C₁₈)-alkoxy, (C₁-C₁₈)-acyloxy, (C₆-C₁₄)-aryl, (C₃-C₁₈)-heteroaryl, (C₂-C₁₇) -heteroalkyl, (C₃-C₈)-cycloalkyl and (C₂-C₁₀)-alkenyl, wherein two adjacent radicals can also be linked to one another to form a ring system and the radicals
R⁵ independently of one another can be (C₁-C₁₈)-alkyl, (C₆-C₁₈)-aryl, (C₆-C₁₈)-aryl-(C₁-C₈)-alkyl, (C₃-C₁₈)-heteroaryl, (C₃-C₁₈)-heteroaryl- (C₁-C₈)-alkyl, (C₂-C₁₇)-heteroalkyl, (C₃-C₈)-cycloalkyl, (C₃-C₈) -cycloalkyl- (C₁-C₈)-alkyl or (C₂-C₁₀)-alkenyl, which can carry one or more (C₁-C₄)-alkyl substituents and the radical
R² can be H or a (C₁-C₈) -alkyl radical, (C₆-C₁₈) -aryl or (C₆-C₁₈)-aryl- (C₁-C₈)-alkyl radical and wherein the radical
R¹¹ can be a (C₁-C₁₈)-alkyl, (C₆-C₁₈)-aryl or (C₆-C₁₈)-aryl-(C₁-C₈)-alkyl radical,
comprising the following process steps:
a) coupling of chiral ferrocenyl sulfoxide with an aromatic aldehyde of the formula (IV) 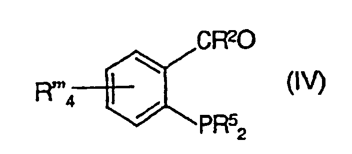 wherein the chiral ferrocenyl sulfoxide is lithiated in the presence of a lithium base and the coupling of the aromatic aldehyde is then carried out by transmetallization in the presence of a metal catalyst of subgroup 8,
b) coupling of the free OH group on the chiral centre of the reaction product from step a) with an organic radical R¹¹ by addition of the corresponding halide R¹¹Hal in the presence of an alkali metal hydride and
c) substitution of the sulfoxide group of the reaction product from step b) by a phosphohalide of the formula HalPR⁵₂ in the presence of a strong lithium base.

10. Process according to claim 9, **characterized in that** before the further reaction, the diastereomers obtained from step a) and/or the diastereomers from step b) are separated.

## Revendications

1. Ligands de ferrocényle de formule générale (II) : 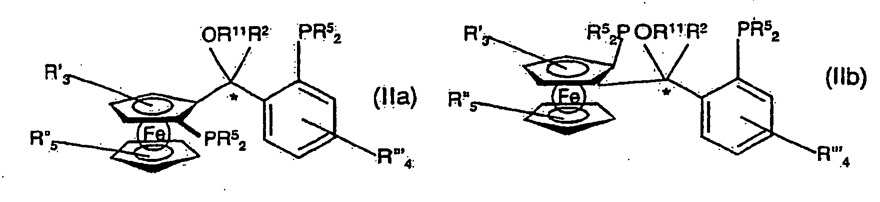 **caractérisés en ce que**
l'énantiomère *S*_{*fc*}*,S* de formule (IIIa) dans le mélange de stéréoisomères (IIa) ou l'énantiomère *R*_{*fc*}*,R* de formule (IIIb) dans le mélange de stéréoisomères (IIb) 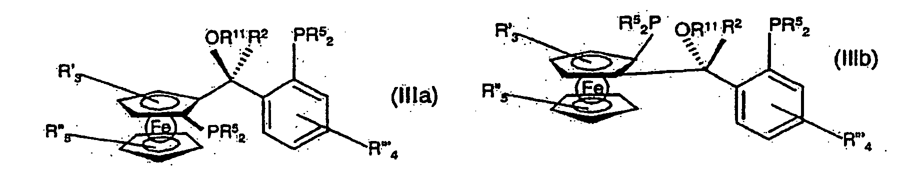 est renforcé et
R' et R" représentent des radicaux qui peuvent être choisis indépendamment l'un de l'autre dans le groupe constitué par H ou CH₃ ou qui peuvent représenter un groupe de liaison, qui relie le ligand avec un support polymère, et les radicaux
R''' représentent des radicaux qui peuvent être choisis indépendamment l'un de l'autre dans le groupe constitué par H ou alkyle en C₁ à C₄, et les radicaux
R⁵ représentent indépendamment l'un de l'autre un radical aryle en C₆, cycloalkyle en C₅ à C₆, adamantyle ou alkyle en C₁ à C₄ qui peuvent porter un ou plusieurs substituants alkyle en X₁ à C₄ et
R² représente un hydrogène ou un radical alkyle en C₁ à C₄ et
R¹¹ représente un radical alkyle en C₁ à C₄.

2. Ligands de ferrocényle selon la revendication 1,
**caractérisés en ce que**
R¹¹ est un radical méthyle et/ou
R² représente H ou un radical méthyle et/ou
R', R" et R'" représentent des radicaux hydrogène et/ou
les radicaux R⁵ représentent indépendamment l'un de l'autre des radicaux phényle, toluyle ou xylyle.

3. Ligands de ferrocényle selon l'une des revendications 1 ou 2, **caractérisés en ce que** l'énantiomère *S*_{*fc*}*,S* ou l'énantiomère *R*_{*fc*}*,R* dans le mélange de stéréoisomères représente une fraction supérieure à 60 %.

4. Ligands de ferrocényle selon d'une des revendications 1 à 3, **caractérisés en ce que** le ligand se présente sous forme d'énantiomère *S*_{*fc*}*,S* ou sous forme d'énantiomère *R*_{*fc*}*,R* avec une pureté supérieure à 99 %.

5. Utilisation de ligands de ferrocényle selon d'une des revendications 1 à 4, pour la production de composés complexes.

6. Utilisation de ligands de ferrocényle selon la revendication 5, pour la production de composés complexes avec des métaux, des sels métalliques ou des précomplexes métalliques des 7^{ème} ou 8^{ème} groupe secondaire de la classification périodique des éléments.

7. Utilisation de ligands de ferrocényle selon d'une des revendications 1 à 4, dans l'hydrogénation ou l'hydroformylation asymétrique d'un composé organique insaturé.

8. Utilisati on de ligands de ferrocényle selon la revendication 7, dans l'hydrogénation asymétrique de liaisons C=C, C=O- ou C=N.

9. Procédé de production de ligands de ferrocényle de formule générale (II) : 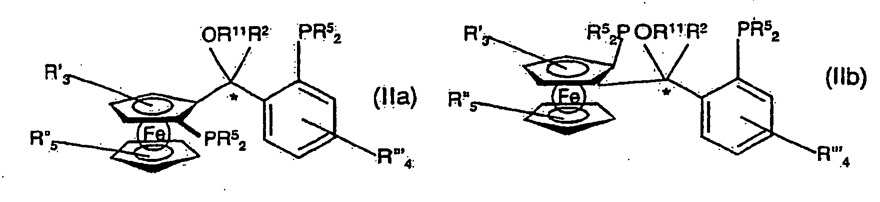 **caractérisés en ce qu'**
on renforce l'énantiomère *S*_{*fc*}*,S* de formule (IIIa) dans le mélange de stéréoisomères (IIa) ou l'énantiomère *R*_{*fc*}*,R* de formule (IIIb) dans le mélange de stéréoisomères (IIb) 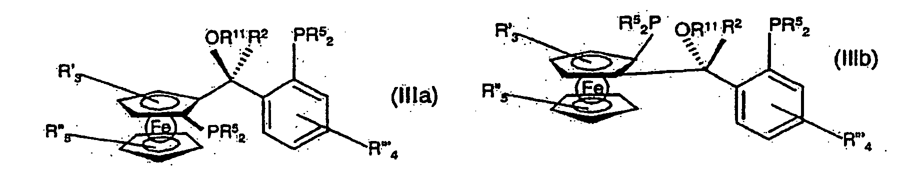 et
R' et R" représentent des radicaux qui peuvent être choisis indépendamment l'un de l'autre dans le groupe constitué par H ou CH₃ ou qui peuvent représenter un groupe de liaison qui relie le ligand avec un support polymère, et les radicaux
R''' représentent des radicaux qui peuvent être choisis indépendamment l'un de l'autre dans le groupe constitué par H, alkyle en C₁ à C₁₈, alcoxy en C₁ à C₁₈, acyloxy en C₁ à C₁₈, aryle en C₆ à C₁₄, hétéroaryle en C₃ à C₁₈, hétéroalkyle en C₂-C₁₇, cycloalkyle en C₃ à C₈, alcényle en C₂ à C₁₀, deux radicaux voisins pouvant également être reliés entre eux pour former un système cyclique, et les radicaux
R⁵ peuvent représenter indépendamment l'un de l'autre un alkyle en C₁ à C₁₈, un aryle en C₆ à C₁₈, un aryle (C₆-C₁₈)-alkyle (C₁-C₈), un hétéroaryle en C₃ à C₁₈, un hétéroaryle (C₃ à C₁₈)-aklyle (C₁-C₈), un hétéroalkyle en C₂ à C₁₇, un cycloalkyle en C₃ à C₈, un cycloalkyle (C₃-C₈)-alkyle (C₁-C₈), un alcényle en C₂ à C₁₀, qui peuvent porter un ou plusieurs substituants alkyle en C₁ à C₄, et le radical
R² représente un hydrogène ou un radical alkyle en C₁ à C₈, aryle en C₆ à C₁₈, aryle en (C₆ à C₁₈), alkyle (C₁ à C₈), et le radical
R¹¹ peut représenter un alkyle en C₁ à C₁₈, un aryle en C₆ à C₁₈, un aryle (C₆-C₁₈)-alkyle (C₁-C₁₈),
le procédé comprenant les étapes de procédé suivantes :
a) couplage de ferrocényle-sulfoxyde chiral avec un aldéhyde aromatique de formule (IV), 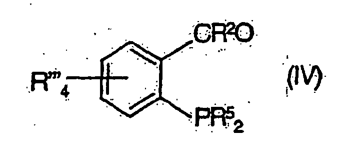 le ferrocényl-sulfoxide chiral étant lithié en présence d'une base de lithium, puis l'aldéhyde aromatique étant couplé par transmétallation en présence d'un catalyseur métallique du 8^{ème} groupe secondaire de la classification périodique des éléments,
b) couplage du groupe OH libre sur le centre chiral du produit de réaction de l'étape a) avec un radical organique R¹¹ par addition d'un halogénure R¹¹Hal, de préférence l'iodure R¹¹I, en présence d'un hydrure de métal alcalin, et
c) substitution du groupe sulfoxyde du produit réactionnel de l'étape b) en présence d'une base de lithium forte par un phosphohalogénure de formule HalPR⁵₂.

10. Procédé selon la revendication 9,
**caractérisé en ce qu'**
avant la poursuite de la réaction on sépare les diastéréomères obtenus de l'étape a) et/ou les diastéréomères de l'étape b).
